# EUROPEAN PATENT APPLICATION

(11) **EP 1 698 899 A1**
(43) Date of publication of application: **06.09.2006**
(21) Application number: 04817413.0
(22) Date of filing: 25.10.2004
(51) Int. Cl.: G01N 33/574, C07K 16/18, C12Q 1/02

(54) **DIAGNOSTIC FOR UTERINE GLAND CANCER AND METHOD OF DETECTING GLAND CANCER CELL**

(30) Priority: 30.10.2003 JP 2003370216
(71) Applicant: SYSMEX CORPORATION, Kobe-shi, Hyogo 651-0073 (JP)
(72) Inventor: NAKANO, Koichi, c/o Sysmex Corporation, Kobe-shi, Hyogo 651-0073 (JP); WATANABE, Miharu, c/o Sysmex Corporation, Kobe-shi, Hyogo 651-0073 (JP)
(74) Representative: HOFFMANN EITLE
(86) International application number: PCT/JP2004/015784
(87) International publication number: WO 2005/043166

(57) **Abstract**

The present invention provides diagnostic reagent or reagent kit whereby the presence of adenocarcinoma cells can be detected in cells obtained from uterine cervix obtained for cytodiagnosis, in particular, the presence of adenocarcinoma can be specifically detected in distinction from squamous carcinoma cells; and methods of detecting adenocarcinoma cells. Adenocarcinoma cells are detected by determining cytokeratin 8 with use of cytokeratin 8 antibody optionally with its secondary antibody. Cytokeratin 8 is contained in the largest amount in adenocarcinoma cells among cells obtained from uterine cervix. The detection sensitivity is increased by employing HIK 1083 antibody together. The subject specimen may comprise either uterine cervix tissues or individually dispersed cells.

## Description

### Technical Field

The present invention relates to diagnostic reagent for uterine adenocarcinoma whereby the presence of an adenocarcinoma cell is specifically detected from cells obtained from uterine cervix; and methods of detecting adenocarcinoma cells.

### Background Art

Uterine cervical cancer is classified into squamous carcinoma and adenocarcinoma, and 95% of uterine cervical cancer is said to be squamous carcinoma, however, the incidence of adenocarcinoma is increasing in recent years. There is high incidence of adenocarcinoma in relatively young patients and it gives a clinically serious problem because of its poor prognosis (Lea JS, Sheets EE, Wenham RM, Duska LR, Coleman RL, Miller DS, Schorge JO. Gynecol Oncol. 2002 ;84(1):115-119.).

In general cytodiagnostic method in which cells are stained with Papanicolaou staining and grade of malignancy is determined from their cell morphology, it is difficult to find adenocarcinoma because the cytologic feature of a normal glandular cell and the one of a malignant glandular cell are similar (Channg WC, Matisic JP, Zhou C, Thomson T, Clement PB, Hayes MM.Cancer.1999. 87(1):5-11.) (Gallup DG, Harper RH, Stock RJ. Gynecol. 1985; 65(3): 416-422.)
Therefore, adenocarcinoma is often missed, and there are many cases with high suspicion of adenocarcinoma from the clinical condition and local findings, despite the negative result obtained in cytodiagnosis.

In histopathological diagnosis, disorganized cell arrangement caused by cancer cells can be observed and the possibility of detecting cancers is higher than the one in cytodiagnosis. However, obtaining a biopsy specimen is accompanied by pain. Therefore a biopsy is not applicable to examinations such as a general medical check.

On the other hand, if there is a specific marker for cancer cells, more accurate diagnosis is possible even in cytodiagnosis. Concerning uterine cancer, SCC (squamous cell carcinoma) antigen has been conventionally well known as a tumor marker.
SCC antigen is derived from squamous carcinoma, and SCC antibody against SCC antigen is effective for detection of squamous carcinoma. However, SCC antibody has a low ratio of positive against adenocarcinoma and it is not specific for adenocarcinoma. Therefore, adenocarcinoma can not be distinguished from cervical squamous carcinoma by using the antibody. Furthermore, since positive ratio in detecting early stage cancer with SCC antibody is not high either, the antibody is far from effective as a diagnostic for examination with the main purpose of early detection of cancer.

Alternatively, it is proposed that cytokeratin of filamentous proteins which exist within cells is used as a marker and cytokeratin antibody which can bind directly to keratin marker is utilized as a diagnostic (Patent Reference 1).

For example, Non-Patent Reference 1 discloses detection of colon carcinoma with use of cytokeratin 8 antibody. In Patent Reference 2, reagents for detecting uterine cancer by employing monoclonal antibody which binds to cytokeratin 19 are disclosed. However, cytokeratin 19 antibody reacts not only with adenocarcinoma cells but also with squamous carcinoma, therefore, the antibody can not be used to distinguish adenocarcinoma from cervical squamous carcinoma.

Patent reference 1: U.S. Pat. No. 4,775,620
Patent reference 2: U.S. Pat. No. 5,288,614
Non-Patent Reference 1: Makin CA, Bobrow LG, Bodmer WF. Monoclonal antibody to cytokeratin for use in routine histopathology. J Clin Pathol. 1984 ;37(9):975-983.

### Disclosure of the Invention

### Problems to be resolved by the invention

The present invention was made in view of the circumstances described above, and an object of the present invention is to provide a diagnostic reagent or reagent kit whereby the presence of adenocarcinoma cells can be detected in cells obtained from uterine cervix obtained for cytodiagnosis, in particular, the presence of adenocarcinoma can be specifically detected in distinction from cervical squamous carcinoma cells; and methods of detecting adenocarcinoma cells.

### Means of Solving the Problems

The diagnostic reagent for adenocarcinoma of the present invention is a diagnostic regent for determining the presence or absence of adenocarcinoma in a specimen of cells obtained from uterine cervix. The reagent comprises cytokeratin 8 antibody. The reagent may further comprise HIK1083 antibody.

The diagnostic reagent kit for uterine adenocarcinoma of the present invention comprises a first reagent containing cytokeratin 8 antibody, and a second reagent containing a labeled secondary antibody which can bind to cytokeratin 8 antibody.

The detecting method of the present invention is a method of detecting an adenocarcinoma cell in a specimen of cells obtained from uterine cervix. The method comprises a step of allowing cytokeratin 8 antibody to react with the cells. The method may further comprise the steps of removing a solution containing unreactant to collect cells, and detecting a complex of the cytokeratin 8 antibody and the adenocarcinoma cell in the cells collected. The specimen may comprise a uterine cervix tissue or cells dispersed individually.

The distinguishing method of the present invention is a method of distinguishing an adenocarcinoma cell in a specimen of cells obtained from uterine cervix including an adenocarcinoma cell and a squamous carcinoma cell. The method comprises the steps of allowing a labeled cytokeratin 8 antibody to react with the cells, and detecting a complex of the cytokeratin 8 antibody and the adenocarcinoma cell.

### Effect of the Invention

The diagnostic reagent or reagent kit of the present invention comprises cytokeratin 8 antibody which can bind to cytokeratin 8, a marker specific for adenocarcinoma cells. Therefore, the reagent or reagent kit can distinguish adenocarcinoma cells from normal glandular cells, regardless of their similar cell morphology. Furthermore, the reagent or reagent kit can distinguish adenocarcinoma cells from cervical squamous carcinoma cells. Accordingly, by using the reagent or reagent kit of the present invention, adenocarcinoma cells can be specifically detected not only from tissues to be diagnosed but also from cell population substantially comprising of individually dispersed cells (i.e. single cells).

### Brief Description of Drawings

Fig. 1 is a fluorescence micrograph (×200) of a tissue specimen containing cervical adenocarcinoma.
Fig. 2 is a micrograph (×200) of a hematoxylin-eosin stained tissue specimen comprising cervical adenocarcinoma.
Fig. 3 is a fluorescence micrograph (×200) of a normal squamous epithelial tissue of uterine cervix.
Fig. 4 is a fluorescence micrograph (×200) of a normal glandular tissue of uterine cervix
Fig. 5 is a fluorescence micrograph (×400) of adenocarcinoma cells (HeLa cells) derived from uterine cervix.
Fig. 6 is a fluorescence micrograph (×400) of cervical squamous carcinoma cells (C33A cells) derived from uterine cervix.
Fig. 7 is a fluorescence micrograph (×400) of oral squamous cells.

### Best Mode for Carrying Out the Invention

According to the present invention, the diagnostic reagent for adenocarcinoma of uterine cervix comprises cytokeratin 8 antibody.
Cytokeratin is one of the intracellular filamentous components and belongs to a group of proteins forming intermediate filaments in the components. Cytokeratin 8 is contained in an adenocarcinoma cell as well as in a squamous carcinoma cell, a normal glandular cell, and a normal squamous cell, but the content of cytokeratin 8 is overwhelmingly highest in an adenocarcinoma cell. Accordingly, since cytokeratin 8 antibody binds preferentially and specifically to an adenocarcinoma cell, the antibody can distinguish an adenocarcinoma cell from a normal cell and a squamous carcinoma cell.

The cytokeratin 8 antibody used in the present invention may be any antibodies which have binding sites for cytokeratin 8, and the structure of Fc fragment and constant regions of Fab fragment are not particularly limited. For example, "monoclonal mouse anti-human cytokeratin 8 antibody 35H11" commercially available from DakoCytomation may be used.

The diagnostic reagent for adenocarcinoma of the present invention preferably contain saline, phosphate buffer or the like as a solvent for cytokeratin 8 antibody, in particular, phosphate buffered saline (PBS) or PBS containing surfactant (e.g. PBST) is preferably used.

The diagnostic reagent of the present invention may contain HIK1083 antibody besides cytokeratin 8 antibody. Adenocarcinoma of uterine cervix can be detected by using only cytokeratin 8 antibody. However, especially when a specimen contains a large amount of mucus, it may be difficult for cytokeratin 8 antibody to react with the specimen. In this respect, HIK 1083 antibody reacts specifically with mucus (specific kind of mucin) produced by ovary carcinoma and uterine cervical adenocarcinoma. Therefore, by a combined use of cytokeratin 8 antibody and HIK 1083 antibody, adenocarcinoma of uterine cervix can be detected with almost 100% accuracy.

In the diagnostic reagent of the present invention, in order to detect adenocarcinoma cells by using cytokeratin 8 as a marker, cytokeratin 8 antibody itself may be labeled, or a secondary antibody which binds to the cytokeratin 8 antibody as a primary antibody may be labeled. In the latter case, the diagnostic reagent of the present invention corresponds to a diagnostic kit comprising a combination of a first reagent containing cytokeratin 8 antibody and a second reagent containing the labeled secondary antibody.

Labels for the primary antibody (cytokeratin 8 antibody) and the secondary antibody are not particularly limited, and conventionally known labels such as fluorescent substances, radioactive substances and enzymes may be used.

In order to increase the specificity of the reaction between cytokeratin 8 antibody and adenocarcinoma cells in using the diagnostic reagent or reagent kit of the present invention, the surface of a tissue specimen is preferably covered with non-specific proteins (e.g. serum) in advance. Accordingly, it is preferable to use the inventive diagnostic reagent or reagent kit in combination with a blocking reagent to block non-specific reaction with proteins other than the target protein of cytokeratin 8 antibody.

Examples of the blocking reagent used in the present invention include non-specific proteins such as serum, IgG, BSA, skim milk, and it may be appropriately diluted with saline, etc.

The method of detecting adenocarcinoma cells of the present invention is described below.
According to the present invention, the method of detecting adenocarcinoma cells comprises a step of allowing a specimen to react with cytokeratin 8 antibody.

The specimen applied in the method comprises cells obtained from uterine cervix. The specimen may comprise a tissue obtained from uterine cervix or a cell population of cells dispersed individually (i.e. a population of single cells substantially). The tissue does not need to be treated for removing mucus, but it is preferable that the cell population is treated to remove mucus.

The method of removing mucus is not particularly limited, however, the method is preferably implemented not to affect the cell morphology, etc. For example, as the present inventors have proposed, the method of removing mucus by using cysteine and/or a compound derived therefrom may be applied.

Examples of cysteine and/or a compound derived therefrom include methyl cysteine, acetyl cysteine, L-cysteine and the like, and examples of the solvent for cysteine and/or a compound derived therefrom include water, saline, buffer solutions such as PBS (phosphate buffered saline), Tris, etc. The content percentage of cysteine and/or a compound derived therefrom is preferably 5 mass%, or more, more preferably 10 to 20 mass%. When under 5 mass% of cysteine and/or a compound derived therefrom is contained, removing mucus can not be sufficiently performed.

For the methods to disperse cells into individual cells, it is preferable to disperse cells without damaging the morphology of cells. For example, it is preferable to use the method proposed by the present inventors wherein cells are stabilized with an aldehyde compound, etc. and then treated with protease.

For the aldehyde compound, paraformaldehyde, formaldehyde, glutaraldehyde or a mixture of those aldehydes may be used. A solvent for the aldehyde compound may be any solvent which does not react with an aldehyde group in the compound. Preferable solvent is water such as distilled water, ion-exchange water and purified water. Other component which may preferably be contained in the diagnostic reagent is about from 0.1 to 0.4 mass% picric acid. Picric acid may enhance tissue permeability of the aldehyde compound.

Examples of the protease include trypsin, pronase, pepsin, elastase, collagenase and the like. They may be used individually or in a combination of more than two kinds of proteases.
Among these proteases, collagenase is used preferably. When a protease with weak digestive power is used, the time required for dispersion can be determined within relatively wide range, therefore the difference of appropriate digestion time required for each specimen is not problematic. Kinds of collagenases are not particularly limited, and the collagenases may be derived from animals or bacteria, and the substrate specificities of collagenases derived from animals are not particularly limited. Solvents for proteases may be solvents which do not denature proteases. It is preferable to use water such as distilled water, ion-exchange water and purified water for the solvent. The concentration of the protease in the diagnostic reagent is appropriately decided according to the kind of protease. In the case of a protease which has high power of digestion, such as trypsin, the concentration of the protease is preferably about 0.05 to 0.1 mass %, and in the case of collagenase, the preferable concentration is about 0.1 to 1.0 mass %.

Before allowing the specimen to react with cytokeratin 8 antibody, a blocking reagent is preferably used in the pretreatment to block some proteins in the specimen, which may cause non-specific reactions.

Examples of the blocking reagent include serum, IgG, BSA, skim milk and the like.

The specimen after blocking its non-specific reaction is allowed to react with cytokeratin 8 antibody. In the case of a tissue specimen, it is only necessary to keep a solution containing cytokeratin 8 antibody on the surface of the specimen for about 30 minutes in the condition that the antibody can contact with the tissue in the specimen.

When a specimen is a population of dispersed cells, the reaction is performed by mixing the cell population with a solution containing cytokeratin 8 antibody to disperse cells uniformly in the solution.
After the reaction, the excess solution containing the antibody is removed.

When cytokeratin 8 antibodies are labeled, unreacted cytokeratin 8 antibodies are removed and then cytokeratin 8 antibodies bound to adenocarcinoma cells may be detected based on the label of the cytokeratin 8 antibody.

On the other hand, when cytokeratin 8 antibodies are not labeled, it is necessary to react with labeled second antibodies additionally. By performing a reaction with the labeled second antibody which has a binding site with cytokeratin 8 antibody, a complex of cytokeratin 8 antibody and adenocarcinoma cell is detected based on the label.

Labels which may be used in the present invention include, but are not particularly limited to, fluorescent substances such as cyanine dyes like Cy3® (Amersham Life Science Inc.) etc., fluorescein isothiocyanate (FITC), rhodamine, and quantum dot; light scattering substances such as gold particle; light absorptive substances such as ferrite; radioactive substances such as iodine-125 (¹²⁵I); enzymes such as peroxidase, alkali phosphatase, DAB (3,3'-diamino-benzidine tetrahydrochloride and glucose oxidase.

After the reaction with the labeled second antibodies, excess labeled second antibodies are removed and observation is performed in the way suitable for detecting the label. For example, when fluorescent label is used, fluorescence may be detected. In addition, it is preferred to wash the specimen appropriately between each process.

The detection method of the present invention may further comprise a process for the reaction with HIK 1083 antibody. Cytokeratin 8 antibody is an antibody against cytoskeleton and hardly reacts with some cells producing mucus. On the other hand, HIK 1083 antibody reacts specifically with mucus produced by cancer cells. Therefore, by using cytokeratin 8 antibody and HIK 1083 antibody together, cancer cells may be detected with 100% efficiency. Moreover, a combined use of cytokeratin 8 antibody and HIK 1083 antibody enables detection of adenocarcinoma cells with high sensitivity and without being much affected by a condition concerning whether mucus is removed from cells or tissues in the specimen and other conditions.

When both HIK 1083 antibody and cytokeratin 8 antibody are used, the order of the reaction process with cytokeratin 8 antibody and the reaction process with HIK 1083 antibody is not particularly limited. After the reaction with HIK 1083 antibody, the specimen may be washed and then reacted with cytokeratin 8 antibody, or the specimen may be reacted with cytokeratin 8 antibody first, then washed and reacted with HIK1083 antibody. Alternatively, a reagent containing both cytokeratin 8 antibody and HIK 1083 antibody may be used for the reaction with the specimen.

### Examples

### [Specificity of cytokeratin 8 antibody]

### (1) Immunohistochemical assay

For a sample obtained in biopsy of human uterine adenocarcinoma, formalin-fixed and paraffin-embedded tissue section was deparaffinized, and afterwards it was kept still in 100% xylene at room temperature for 10 minutes. Then the tissue section was kept still in 100% ethanol at room temperature for 30 seconds, followed by being kept still in 80% ethanol and then 70% ethanol at room temperature each for 30 seconds. Subsequently, it was kept still in distilled water at room temperature for 30 seconds.

By keeping the tissue section in PBST (PBS (phosphate buffered saline) solution containing 0.05% Tween 20) containing 1% goat serum at room temperature for 10 minutes, non-specific proteins were blocked.
A solution containing cytokeratin 8 antibody (5 µg/ml)(its solvent is PBST containing 1 % goat serum) was gently placed over the sample, and kept still at room temperature for 30 minutes for antigen-antibody reaction.

After the reaction, unreacted antibody solution was removed by aspiration, and the tissue section was washed by shaking in PBST at room temperature for 5 minutes repeatedly for three times.

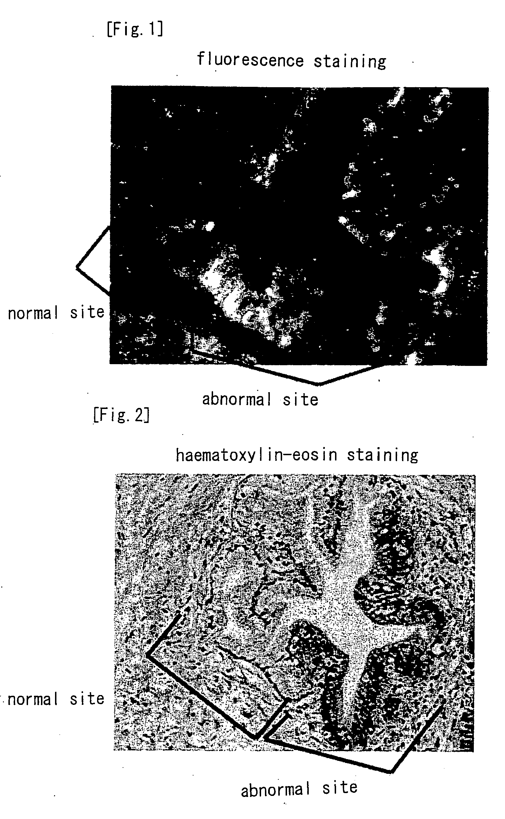

A solution containing F(ab)₂ fragment of goat anti-mouse IgG labeled with fluorescent dye Alexa 488® (Molecular Probes)(5 µg/ml)(its solvent is PBST containing 1% goat serum) was gently placed over the tissue sample for antigen-antibody reaction. After the reaction, solution containing unreacted antibody was removed by aspiration, and the tissue section was washed by shaking in PBST at room temperature for 5 minutes repeatedly for three times. After the washing, PBST containing 50% mounting medium is layered and cover glass was mounted, and then observation was made under a fluorescent microscope to give a photo shown in Fig. 1.

For reference, the same sample obtained in biopsy was stained with haematoxylin-eosin to give a photo in Fig. 2.
As shown in Fig. 2, nuclei became bigger in abnormal sites (adenocarcinoma part). In Fig. 1 which corresponds to Fig.2, it was confirmed that abnormal sites were stained with fluorescence. Accordingly, it is understood that cytokeratin 8 antibody does not react with normal tissue but reacts only with adenocarcinoma part.

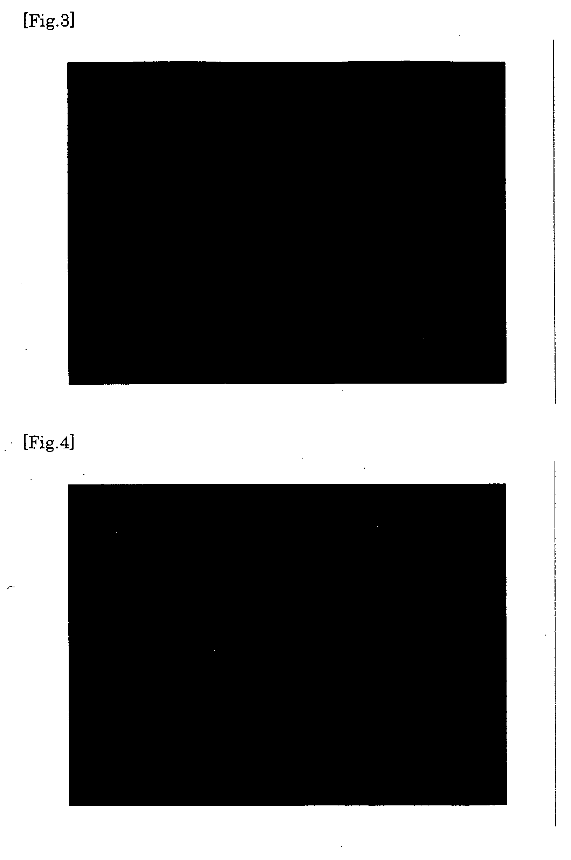

(2) Assay of a normal squamous epithelial tissue and a normal glandular tissue by using cytokeratin 8 antibody The same procedures as the above were conducted for a normal squamous epithelial tissue and a normal glandular tissue by using the sample obtained in biopsy.
A fluorescent micrograph of the normal squamous tissue stained with fluorescence is shown in Fig. 3.
A fluorescent micrograph of the normal glandular tissue stained with fluorescence is shown in Fig. 4.

### (3) Immunocytochemical assay

Cultured normal epithelial cell of oral mucosa as a negative control, C33A which was a cultured cell line derived from cervical squamous carcinoma and HeLa cell which was a cultured cell line derived from cervical adenocarcinoma were used for samples.

After each sample liquid of cell culture was fixed with 100% methanol, it was centrifuged (10000 rpm; 1 minute; 4°C) and the supernatant was removed to collect cells.
To the cells thus collected was added each 1 ml of PBS and PBST for washing. Centrifugation (10000 rpm; 1 minute; 4°C) was conducted and the supernatant was removed to collect cells. 1ml of PBST containing 1% goat serum was added subsequently, and the cell solution was mixed by shaking at room temperature for 10 minutes to block non-specific proteins on the cell surfaces. After the blocking, centrifugation (10000 rpm; 1 minute; 4°C) was performed to remove excess blocking solution.

Then, solution containing cytokeratin 8 antibody (5 µg/ml)(its solvent is PBST containing 1% goat serum) was added and mixed with the cell solution by shaking at room temperature for 30 minutes for antigen-antibody reaction. After the reaction, centrifugation (10000 rpm; 1 minute; 4°C) was performed to remove unreacted antibody solution.
1ml of PBST was added to wash the cells, the supernatant was removed and then the cells were collected.

A solution containing F(ab)₂ fragment of goat anti-mouse IgG, which was labeled with fluorescent dye Alexa 488 (5 µg/ml)(its solvent is PBST containing 1% goat serum) was added and mixed with the cell solution by shaking at room temperature for 30 minutes for antigen-antibody reaction. After the reaction, centrifugation (10000 rpm; 1 minute; 4°C) was performed and the supernatant was removed to collect cells.

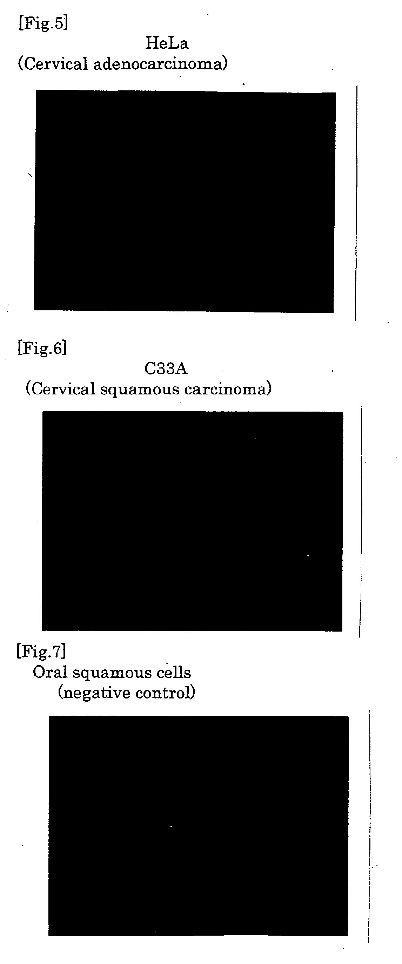

1ml of PBST was added to wash the cells, centrifugation (10000 rpm; 1 minute; 4°C) was performed and the supernatant was removed to collect cells.
The cells were resuspended in PBS containing 50% glycerol and observed under a fluorescent microscope. The results are shown in Fig. 5 to Fig 7.

It was confirmed that cytokeratin 8 antibody does not react with squamous cells in Fig. 6 and Fig. 7. On the other hand, fluorescent part was observed for uterine cervix adenocarcinoma cells and it was proved that cytokeratin 8 antibody reacts with adenocarcinoma cells (Fig. 5).

### Industrial Applicability

The diagnostic reagent or reagent kit for adenocarcinoma of the present invention can specifically detect adenocarcinoma in uterine cervix tissue as well as a population of individually dispersed cells derived from uterine cervix. Therefore, the reagent or reagent kit can be utilized for diagnosis for uterine cervical glandular cancer, especially for distinguishing adenocarcinoma from squamous cell carcinoma. According to the present invention, the method of detecting adenocarcinoma cells can be employed when adenocarcinoma is required to be detected specifically from uterine cervix tissue as well as a population of individually dispersed cells derived from uterine cervix.

## Claims

1. A diagnostic reagent for determining the presence or absence of adenocarcinoma in a specimen of cells obtained from uterine cervix, said diagnostic reagent comprising cytokeratin 8 antibody.

2. The diagnostic reagent according to claim 1, further comprising HIK1083 antibody.

3. A diagnostic reagent kit for uterine adenocarcinoma comprising:
a first reagent containing cytokeratin 8 antibody; and
a second reagent containing a labeled secondary antibody which can bind to cytokeratin 8 antibody.

4. A method of detecting an adenocarcinoma cell in a specimen of cells obtained from uterine cervix, said method comprising a step of allowing cytokeratin 8 antibody to react with the cells.

5. A method of detecting an adenocarcinoma cell in a specimen of cells obtained from uterine cervix, said method comprising the steps of:
allowing a reagent containing cytokeratin 8 antibody to react with the cells;
removing a solution containing unrcactant to collect cells; and
detecting a complex of the cytokeratin 8 antibody and the adenocarcinoma cell in the collected cells.

6. The method according to claim 4, wherein the specimen comprises a uterine cervix tissue or cells dispersed individually.

7. The method according to claim 5, wherein the specimen comprises a uterine cervix tissue or cells dispersed individually.

8. A method of distinguishing an adenocarcinoma cell in a specimen of cells obtained from uterine cervix including an adenocarcinoma cell and a squamous carcinoma cell, said method comprising the steps of:
allowing a labeled cytokeratin 8 antibody to react with the cells; and
detecting a complex of the cytokeratin 8 antibody and the adenocarcinoma cell.
